(19) Europäisches Patentamt
European Patent Office
Office européen des brevets

(11) **EP 3 239 285 B1**

(12) **EUROPÄISCHE PATENTSCHRIFT**

(45) Veröffentlichungstag und Bekanntmachung des Hinweises auf die Patenterteilung:
**30.01.2019 Patentblatt 2019/05**

(51) Int Cl.:
*C12M 1/32* (2006.01)  *C12M 1/00* (2006.01)
*C12M 1/34* (2006.01)  *C12M 1/36* (2006.01)

(21) Anmeldenummer: **16167322.3**

(22) Anmeldetag: **27.04.2016**

(54) **VORRICHTUNG ZUR ERMITTLUNG UND ÜBERWACHUNG DER PHYSIOLOGISCHEN ZUSTÄNDE VON MIKROBIELLEN KULTUREN IN JEDEM EINZELNEN MIKROBIOREAKTOR EINER MIKROTITERPLATTE**

DEVICE FOR DETERMINING AND MONITORING THE PHYSIOLOGICAL STATES OF MICROBIAL CULTURES IN EACH MICRO-BIOREACTOR OF A MICROTITRATION PLATE

DISPOSITIF DE DETERMINATION ET DE SURVEILLANCE DE L'ETAT PHYSIOLOGIQUE DE CULTURES MICROBIENNES DANS CHAQUE MICRO-BIOREACTEUR D'UNE PLAQUE DE MICROTITRATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**01.11.2017 Patentblatt 2017/44**

(73) Patentinhaber: **Rheinisch-Westfälische Technische Hochschule (RWTH) Aachen 52056 Aachen (DE)**

(72) Erfinder:
• **Büchs, Prof. Dr. Ing. Jochen**
**52064 Aachen (DE)**
• **Flitsch, David**
**52072 Aachen (DE)**
• **Wandrey, Georg**
**52074 Aachen (DE)**
• **Kunze, Martin**
**72108 Rottenburg (DE)**
• **Giese, Dr. Ing. Heiner**
**42699 Solingen (DE)**

(74) Vertreter: **Kohlmann, Kai Donatusstraße 1 52078 Aachen (DE)**

(56) Entgegenhaltungen:
EP-B1- 0 905 229  WO-A1-2009/039433
WO-A1-2014/130357  DE-A1-102014 012 246
DE-C2- 4 415 444  US-A1- 2007 275 455
US-A1- 2015 036 125

• BUCHS NICOLAS C ET AL: "Safety of robotic general surgery in elderly patients.", JOURNAL OF ROBOTIC SURGERY AUG 2010, vol. 4, no. 2, August 2010 (2010-08), pages 91-98, ISSN: 1863-2483

**Beschreibung**

Die Erfindung betrifft eine Vorrichtung zur Ermittlung und Überwachung des physiologischen Zustandes von mikrobiellen Kulturen in jedem einzelnen Mikrobioreaktor einer Mikrotiterplatte.

Aus der DE 44 15 444 C2 ist ein Verfahren und eine Vorrichtung zur Ermittlung und Überwachung des physiologischen Zustandes mikrobieller Kulturen durch quasi kontinuierliche Messung der Atmungsaktivitäten in einem geschüttelten Messkolben unter sterilen Bedingungen bekannt, wobei der Messkolben in einer Spülphase zur Versorgung der mikrobiellen Kulturen mit Spülgas durchströmt wird, anschließend in einer Messphase der Spülgasstrom in den Gasraum des Messkolbens mit mindestens einem Absperrmittel unterbrochen und mit mindestens einem Messmittel mindestens eine für die Atmungsaktivität aussagekräftige Messgröße erfasst wird, die nach Umsetzung in ein elektrisches Signal in einem Steuerrechner verarbeitet wird. Um den zeitlichen Verlauf von Kultivierungen aerober Mikroorganismen in den Messkolben zu verfolgen und zu kontrollieren wird quasi kontinuierlich die Sauerstofftransferrate (OTR) unter sterilen Bedingungen erfasst. In der Messphase wird der Gasraum in dem Messkolben über das mindestens eine ansteuerbare Absperrmittel abgesperrt und der Abfall des Sauerstoffpartialdrucks mit einer sterilisierbaren $pO_2$-Elektrode gemessen. Ein Ausfall dieser $pO_2$-Elektrode zwingt zum Abbruch des Versuchs, da sie nicht gewechselt werden kann, ohne die Kultur zu kontaminieren. Außerdem erfordert das aus der DE 44 15 444 C2 bekannte Verfahren den Einsatz sterilisierbarer Messwertgeber.

In der EP 0 905 229 B1 wird daher vorgeschlagen, jede Messgröße durch ein steriles Trennmittel zwischen dem Gasraum des Messkolbens und dem Messwertgeber hindurch zu erfassen. Zum Auswechseln des Messwertgebers wird dieser von dem Messkolben gelöst, während das Trennmittel am Messkolben verbleibt.

Die US 2007/275455 A1 offenbart eine Vorrichtung für das Handhaben von mehreren Platten, in denen Zellkulturen durchgeführt werden. Die Platten werden von einem Roboterarm auf einen Mikroskoptisch bewegt. Jede Platte besitzt achtzehn Reservoire, wobei in drei Reservoiren Zellsuspensionen oder Nährmedien als Flüssigkeiten vorgehalten werden. Die drei Reservoire sind über mikrofluidische Kanäle mit Mikrobioreaktoren verbunden. Die übrigen Reservoire dienen zur Steuerung der den einzelnen Mikrobioreaktoren zugeordneten Ventile. Die mikrofluidischen Ventile dienen dazu, Zellsuspensionen oder Nährmedien in die Mikrobioreaktoren ein- bzw.- auszuleiten, um die einzelnen Zellen unter einem Mikroskop zu beobachten.

Aus der DE 10 2014 012 246 A1 ist eine Vorrichtung und ein Verfahren zur Durchführung von Batch-Biogasreaktionen mithilfe eines Mikrotiter-Plattentests bekannt. Die Inkubation kann ruhend oder geschüttelt durchgeführt werden. Die Menge an gebildetem Biogas wird mittels eines miniaturisierten Drucksensors in den einzelnen Kavitäten der Mikrotiterplatte erfasst.

Weiter sind aus dem Stand der Technik Mikrotiterplatten als Hochdurchsatz-Systeme zur Kultivierung mikrobieller Kulturen bekannt. Mikrotiterplatten haben in der jüngeren Vergangenheit in vielen Gebieten den klassischen Schüttelkolben als Kultursystem verdrängt. Aufgrund der fortschreitenden Miniaturisierung sind eine Vielzahl von Kultivierungen parallel durchführbar. Die in Mikrotiterplatten untersuchten Probenvolumina sind geringer, was Ressourcen und Kosten spart.

Ausgehend von diesem Stand der Technik liegt der Erfindung die Aufgabe zugrunde, eine Vorrichtung zur Ermittlung und Überwachung des physiologischen Zustandes von mikrobiellen Kulturen in den Mikrobioreaktoren (Wells) einer Mikrotiterplatte zu schaffen, bei der die Kultivierung in den einzelnen Mikrobioreaktoren unter übereinstimmenden Versuchsbedingungen durchführbar ist.

Diese Aufgabe wird durch eine Vorrichtung zur Ermittlung und Überwachung des physiologischen Zustandes von mikrobiellen Kulturen in jedem einzelnen Mikrobioreaktor einer Mikrotiterplatte mit den Merkmalen des Anspruchs 1 gelöst.

Um übereinstimmende Versuchsbedingungen in jedem Mikrobioreaktor der Mikrotiterplatte zu schaffen ist es erforderlich, dass Totvolumen in der Gaszuführung des Spülgases so gering wie möglich zu halten und sämtliche Mikrobioreaktoren der Mikrotiterplatte mit derselben Menge an Spülgas zu versorgen. Zur Reduktion des Totvolumens sind die Absperrmittel zum Unterbrechen des Spülgasstromes unmittelbar an jedem Mikrobioreaktor angeordnet. Hierdurch wird bewirkt, dass nach dem Absperren in der Messphase kein weiterer, unkontrollierter Zufluss von Spülgas in die Mikrobioreaktoren erfolgt. Des Weiteren sind die Strömungswiderstände in der Gasversorgung und der Strömungswiderstand jedes Mikrobioreaktors derart eingerichtet, dass der Spülgasstrom in der Spülphase in sämtlichen Mikrobioreaktoren im technischen Sinne übereinstimmt. Um dieses Ziel zu erreichen, ist der Strömungswiderstand jedes Mikrobioreaktors so hoch, dass die Strömungswiderstände der Gasversorgung, namentlich der Strömungswiderstand der zu den einzelnen Mikrobioreaktoren führenden Zuführ- und Abführleitungen für das Spülgas im technischen Sinne vernachlässigbar ist.

Der Strömungswiderstand jedes Mikrobioreaktors ist vorzugsweise um mindestens den Faktor 100 größer als die Strömungswiderstände in der Gasversorgung. Aufgrund einer derartigen Einstellung der Strömungswiderstände bedarf es keiner individuellen, den Spülgasstrom steuernden Einrichtung für jeden Mikrobioreaktor, um einen im technischen Sinne übereinstimmenden Spülgasstrom in sämtlichen Mikrobioreaktoren zu gewährleisten.

In der an die Spülphase anschließenden Messphase wird, wie bei der quasi kontinuierlichen Messung der Atmungsaktivitäten im geschüttelten Messkolben, mithilfe von Messmitteln in jedem einzelnen Mikrobioreaktor der physiologische Zustand der mikrobiellen Kultur erfasst.

Die Gasversorgung der erfindungsgemäßen Vorrichtung umfasst eine Spülgaseinspeisung und eine Gasverteilung. Als Spülgaseinspeisung kommt beispielsweise mindestens ein Speicher mit Spülgas oder mindestens ein Spülgas führendes Netz in Betracht.

Umfasst die Einspeisung mehrere Speicher oder mehrere Netze, die unterschiedliche Spülgase bereit stellen, kann optional eine Gasmischbatterie zwischen der Spülgaseinspeisung und der Gasverteilung angeordnet sein. Mit der Mischbatterie kann gezielt eines der Spülgase oder eine Mischung unterschiedlicher Spülgase in die Gasverteilung eingespeist werden.

Die Gasverteilung des Spülgases weist vorzugsweise eine zentrale Zuführleitung auf, die sich von der Gaseinspeisung zu einer an der Mikrotiterplatte angeordneten Unterverteilung für das Spülgas erstreckt. Die Unterverteilung weist Zuführleitungen beziehungsweise Kanäle für das Spülgas zu den einzelnen Mikrobioreaktoren sowie Abführleitungen beziehungsweise Abführkanälen zum Ausleiten des Spülgases aus den Mikrobioreaktoren auf.

Als Spülgase kommen insbesondere Luft, Sauerstoff und Kohlendioxid, bevorzugt Mischungen der vorgenannten Gase in Betracht.

Das Vorsehen einer zentralen Zuführleitung für das Spülgas reduziert den apparatetechnischen Aufwand für die Gasversorgung erheblich. Insbesondere kann in der zentralen Zuführleitung ein einziges flusssteuerndes Bauteil, beispielsweise eine Pumpe oder einen Durchflussmengenregler angeordnet sein, um den Spülgasstrom zu regeln, der auf die Mikrobioreaktoren aufgeteilt wird. Der hohe und übereinstimmende Strömungswiderstand jedes Mikrobioreaktors bewirkt, dass sich der Spülgasstrom innerhalb der Unterverteilung in im Wesentlichen gleich große Teilströme aufteilt und jeder einzelne Mikrobioreaktor übereinstimmend mit Spülgas versorgt wird. Aufgrund der übereinstimmenden, deutlich höheren Strömungswiderstände sämtlicher Mikrobioreaktoren gegenüber den Strömungswiderständen in den Zu- und Abführleitungen zu den einzelnen Mikrobioreaktoren müssen diese keine übereinstimmende Länge und/oder keinen übereinstimmenden Querschnitt aufweisen, da die unterschiedlichen Strömungswiderstände der Leitungen zu vernachlässigen sind.

Die zentrale Zuführleitung in der Gasversorgung bietet darüber hinaus die Möglichkeit, eine Waschflasche zur Anfeuchtung des Spülgases zentral für sämtliche Mikrobioreaktoren vorzusehen. Es entspricht den physikalischen Gegebenheiten, dass Fermentationslösungen Feuchtigkeit verlieren, wenn sie mit trockenem Spülgas in Kontakt gelangen. Dieser Feuchtigkeitsverlust kann durch Anfeuchten der Spülgase in Waschflaschen kompensiert werden.

Die an jedem Mikrobioreaktor angeordneten Absperrmittel weisen zumindest ein Einlassventil auf, das einen die Einlassöffnung umgebenden Ventilsitz sowie eine pneumatisch betätigte Absperrmembran zum Öffnen und Schließen der Einlassöffnung umfasst. Vorzugsweise wird der Spülgasstrom in jedem Mikrobioreaktor jedoch durch Ein- und Auslassventile gesteuert. Die pneumatisch betätige Absperrmembran trägt zu einer kompakten Bauform der Ventile bei, die es ermöglicht, die Absperrmittel trotz des geringen zur Verfügung stehenden Raums unmittelbar an jedem Mikrobioreaktor anzuordnen. Darüber hinaus wird das Totraumvolumen aufgrund des Membranprinzips der verwendeten Ventile weiter reduziert. Durch den Werkstoff und die Werkstoffqualität der Absperrmembran können zudem Ablagerungen an dem Schließkörper des Ventils und damit das Risiko einer Kontamination der mikrobiellen Kultur reduziert werden.

Die Einlassventile mehrerer Mirkobioreaktoren lassen sich synchron betätigen, wenn auf der dem Ventilsitz abgewandten Seite der Absperrmembran der Einlassventile eine mit Unter- und/oder Überdruck beaufschlagbare Druckkammer angeordnet ist, die zum gleichzeitigen pneumatischen Betätigen der Absperrmembran mehrerer Einlassventile eingerichtet ist. Insbesondere wird eine Wandfläche der Druckkammer von der Absperrmembran gebildet, die sich in Folge einer Beaufschlagung mit Unter- beziehungsweise Überdruck entweder in das Innere der Druckkammer beziehungsweise nach außen bewegt. Die membrangesteuerten Auslassventile mehrerer Mirkobioreaktoren lassen sich in gleicher Weise synchron mithilfe einer mit Unter- und/oder Überdruck beaufschlagbaren Druckkammer betätigen.

Die Einlassventile und die Auslassventile der Vorrichtung werden vorzugsweise zeitversetzt angesteuert. Zu Beginn der Messphase werden zunächst sämtliche Einlassventile synchron geschlossen. Mit einem Zeitversatz, beispielsweise von einer Sekunde, werden anschließend sämtliche Auslassventile synchron geschlossen. Dieses zeitversetzte Schließen sämtlicher Ein- und Auslassventile stellt sicher, dass in jedem Fall ein Druckausgleich zwischen dem Gasraum jedes Mikrobioreaktors und der umgebenden Atmosphäre stattgefunden hat, bevor der Gasraum vollends abgeschlossen wird. Damit ergibt sich ein eindeutig definierter Bezugsdruck innerhalb des Gasraumes während der Messphase. Am Ende der Messphase werden zunächst sämtliche Auslassventile synchron geöffnet. Mit einem Zeitversatz, beispielsweise von einer Sekunde, werden anschließend sämtliche Einlassventile synchron geöffnet.

Aus konstruktiven Gründen sowie im Interesse der übereinstimmenden Spülgasversorgung sämtlicher Mikrobioreaktoren stimmen die Absperrmittel sämtlicher Mikrobioreaktoren in Anzahl, Art, Größe und Form exakt überein. Der gegenüber der Gasversorgung hohe Strömungswiderstand jedes Mikrobioreaktors wird vorzugsweise durch die von dem Ventilsitz umgebene Einlassöffnung jedes Mikrobioreaktors hervorgerufen. Die Integration des Strömungswiderstandes in den Ventilsitz beziehungsweise die Einlassöffnung trägt den beengten Raumverhältnissen Rechnung.

Insbesondere wird kein Bauraum in der Gasverteilung vor den Absperrmitteln benötigt, um dort einen separaten Strömungswiderstand, beispielsweise in Form einer Drossel vorzusehen.

Zur Vermeidung eines Staudrucks innerhalb der Mikrobioreaktoren während der Spülphase ist in vorteilhafter Ausgestaltung der Erfindung der Querschnitt der Einlassöffnung jedes Mikrobioreaktors kleiner als der Querschnitt der Auslassöffnung jedes Mikrobioreaktors. Der Querschnitt jeder Auslassöffnung ist vorzugsweise mindestens fünf Mal so groß wie der Querschnitt jeder Einlassöffnung.

Die Messmittel sind für eine nach Mikrobioreaktoren aufgelöste Erfassung mindestens einer für die Atmungsaktivität aussagekräftigen Größe der mikrobiellen Kultur in jedem Mikrobioreaktor eingerichtet. In einer bevorzugten Ausführung werden die zeitlichen Partialdruckänderungen von Sauerstoff und Kohlenstoffdioxid aufgrund der Atmungsaktivität erfasst. In einem Messrechner werden der Abfall des Sauerstoffpartialdrucks in die OTR und der Abfall des Kohlenstoffdioxidpartialdrucks in die CTR umgerechnet. In einer vereinfachten Ausführungsform der Erfindung wird lediglich der Abfall des Sauerstoffpartialdrucks erfasst und in dem Messrechner in die OTR umgerechnet.

Die erfindungsgemäße Vorrichtung verwendet vorzugsweise Mikrotiterplatten mit 16 bis 384 Mikrobioreaktoren. Um trotz der beengten Platzverhältnisse in jedem Mikrobioreaktor den physiologischen Zustand der mikrobiellen Kultur erfassen zu können, kommen vorzugsweise passive Messmittel zum Einsatz, die mindestens ein in jedem Mikrobioreaktor angeordnetes passives Messelement umfassen, dessen Messsignal sich durch eine Änderung der Atmungsaktivität ändert.

Der nicht als Baueinheit mit dem passiven Messelement ausgeführte Messwertgeber zum Umformen des Messsignals in elektrische Signale muss dann nicht auf der Mikrotiterplatte angeordnet werden, sondern kann im Abstand von dem Schüttler für die Mikrotiterplatte angeordnet sein. Mithilfe von Übertragungsleitungen werden die Messsignale zwischen jedem passiven Messelement und einem der Messwertgeber übertragen.

Um bei derartigen passiven Messmitteln eine kontaktlose Erfassung der Atmungsaktivität innerhalb jedes Mikrobioreaktors zu ermöglichen, ist jedes passive Messelement vorzugsweise als eine an einer transparenten Fläche des Mikrobioreaktors dauerhaft angeordnete Indikatorschicht ausgebildet, die auf Änderungen der Gaskonzentration im Gasinnenraum mit einer Änderung der ausgesendeten elektromagnetischen Strahlung im optischen Bereich reagiert. Die elektromagnetische Strahlung (Messsignal) wird durch die für die elektromagnetische Strahlung transparente Fläche des Mikrobioreaktors in einen Lichtwellenleiter als Übertragungsleitung eingekoppelt und zu dem Messwertgeber übertragen.

In einer bevorzugten Ausführungsform basiert die Ermittlung der für die Atmungaktivität aussagekräftigen Messgröße auf der Messung einer Verkürzung der Fluoreszenzlebensdauer eines Fluoreszenzfarbstoffes bei Anwesenheit des entsprechenden Analyten. Physikalischer Mechanismus ist das sogenannte "quenching" und wird durch die Stern-Vollmer Gleichung beschrieben.

Um aufwendige zeitaufgelöste Messungen der Fluoreszenzlebensdauer zu umgehen, wird in einer bevorzugten Ausführungsform der Erfindung die Indikatorschicht mittels einer in der Intensität modulierten Lichtquelle vom Messwertgeber bestrahlt. Dieses Anregungslicht wird mit einer festen Frequenz moduliert und über den Lichtwellenleiter von dem Messwertgeber zu der Indikatorschicht übertragen. Die intensitätsmodulierte Anregung des Fluoreszenzfarbstoffs bewirkt, dass man das entstandene Fluoreszenzlicht zeitverschoben mit derselben Frequenz durch den Messwertgeber detektiert. Diese Zeitverschiebung ist direkt proportional zur Lebensdauer der Fluoreszenz und hängt somit von dem vorliegenden Partialdruck des antsprechenden Analyten ab. Die Detektion erfolgt im Messwertgeber mittels einer Photodiode als optoelektronischem Sensor mit vorgelagertem und auf das Fluoreszenzlicht angepassten optischen Filter. Dieser verhindert das Auftreffen von Umgebungslicht einer anderen Wellenlänge oder des an Grenzflächen zurückreflektierten Anregungslichts auf die Photodiode. Damit nicht Umgebungslicht, welches den optischen Filter aufgrund einer ähnlichen Wellenlänge verglichen zum Fluoreszenzlicht passieren kann, fälschlicherweise als Fluoreszenzlicht angenommen wird, wird ein sogenannter Lock-In Verstärker in jedemm Messwertgeber integriert.

Dieser wertet die elektrischen Signale, die durch den Lichteinfall auf der Photodiode entstehen, dahingehend aus, dass sämtliche Signale mit einer abweichenden Frequenz von der modulierten Lichtintensität des Messwertgebers nicht berücksichtigt werden.

Aus diesem Grund wird die Messung nicht durch spektral ähnliches Umgebungslicht beeinflusst, solange die Intensität dieses Umgebungslichtes nicht mit der gleichen Frequenz, wie die des Anregungslichtes, moduliert ist.

Um eine möglichst hohe Datendichte in der Erfassung der für die Atmungsaktivität aussagekräftigen Messgrößen zu realisieren, ist eine parallele Messwerterfassung im Gegensatz zu einer sequentiellen Messwerterfassung zu bevorzugen. Damit sich die Messwertgeber nicht gegenseitig durch ungewollten Fluoreszenzlichteinfang von nahgelegenen Indikatorschichten beeinflussen können, sollten sich die entsprechenden Modulationsfrequenzen der modulierten Lichtquellen der Messwertgeber unterscheiden, damit es den Lock-In Verstärkern der Messwertgeber möglich ist, dieses Licht ebenfalls in der Auswertung der entsprechenden elektrischen Signale der Photodiode zu ignorieren.

Ist die Standardfrequenz mit der die Intensität des Anregungslichtes moduliert wird beispielsweise 4.000 Hz, genügt ein Unterschied von etwa 100 Hz für das Anregeungslicht, das in jeweils zueinander benachbarte Mikrobioreaktoren eingeleitet wird, um eine gegenseitige Beeinflussung der Messwertgeber zu unterbinden.

Um eine maximale Datendichte in der Messphase zu erhalten und damit die Messgenauigkeit zu erhöhen, ist vorzugsweise jedes passive Messelement fest mit einem ihm zugeordneten Messwertgeber über eine Übertragungsleitung verbunden. Dies bedeutet, dass die Messmittel eine übereinstimmende Anzahl an passiven Messelementen und Messwertgebern aufweisen. Sofern es sich bei den Messwertgebern um optoelektronische Bauelemente zur Umformung der Messsignale in elektrische Signale handelt, sind hiermit nicht unerhebliche Kosten verbunden. Mithilfe eines optischen Multiplexers gemäß den Merkmalen des Anspruchs 19 lässt sich ein Kompromiss zwischen der erforderlichen Datendichte und der Anzahl der Messwertgeber finden. Die Lichtwellenleiter sind mit den Eingängen des optischen Multiplexers und die Messwertgeber mit dessen Ausgängen verbunden. Die an unterschiedlichen Eingängen anliegenden Messsignale sind mithilfe des Multiplexers nacheinander zu einem der Ausgänge durchschaltbar. Im Ergebnis teilen sich die passiven Messelemente mehrerer Mikrobioreaktoren mit Hilfe des Multiplexers einen Messwertgeber.

Um die Spülgasversorgung der Mikrobioreaktoren auf dem engen Bauraum der Mikrotiterplatte auszuführen, sind in vorteilhafter Ausgestaltung der Erfindung sämtliche Absperrmittel und die Unterverteilung für das Spülgas in eine Abdeckung integriert, die nach Art eines Deckels auf die Mikrotiterplatte aufsetzbar ist.

Sofern die Absperrmittel als pneumatisch betätigte Ventile mit einer Absperrmembran ausgeführt sind, sind auch die mit Unter- und/oder Überdruck beaufschlagbaren Druckkammern zum Betätigen der Absperrmembran der Ventile in die Abdeckung integriert.

Zwischen der Mikrotiterplatte und der Abdeckung ist vorzugsweise eine Sterilbarriere angeordnet, die sich über die gesamte Oberfläche der Mikrotiterplatte erstreckt. Die Sterilbarriere ist gasdurchlässig und verhindert zugleich eine Kontamination der Mikrobioreaktoren mit Fremdorganismen. Da sich die Absperrmittel sämtlich in der Abdeckung, d.h. auf ein- und derselben Seite der Sterilbarriere befinden, ist abweichend zu der herkömmlichen Schüttelkolbentechnik lediglich noch eine Sterilbarriere pro Mikrobioreaktor erforderlich. In der herkömmlichen Schüttelkolbentechnik sind indes Sterilbarrieren im Einlass, Auslass und vor dem Messmittel angeordnet.

Bauartbedingt liegen bei der erfindungsgemäßen Mikrotiterplatte die Einlass- und Auslassöffnungen an der Oberseite jedes Mikrobioreaktors relativ nahe bei einander. Zwischen der Einlass- und Auslassöffnung jedes Mikrobioreaktors ist daher vorzugsweise eine Barriere derart angeordnet, dass ein Kurzschluss des Spülgasstroms zwischen Ein- und Auslass unterbunden ist.

Die Fertigung der Abdeckung mit integrierten Funktionselementen lässt sich dadurch vereinfachen, dass die Abdeckung aus mehreren, zu einem Stapel miteinander verbundenen Platten zusammengesetzt ist.

Nachfolgend wird die Erfindung anhand der Figuren näher erläutert. Es zeigen

**Figur 1** eine schematische Darstellung einer erfindungsgemäßen Vorrichtung,

**Figur 2** eine schematische Schnittdarstellung durch einen Mikrobioreaktor der erfindungsgemäßen Vorrichtung,

**Figur 3** eine schematische Darstellung pneumatisch betätigter Ventile eines Mikrobioreaktors nach Figur 2 in unterschiedlichen Schaltstellungen

**Figur 4** eine schematische Schnittdarstellung längs der Linien 33, 34 nach Figur 2,

**Figur 5** eine schematische Schnittdarstellung einer Abdeckung für eine Mikrotiterplatte gemäß der vorliegenden Erfindung,

**Figur 6a** eine isometrische Ansicht eines optischen Multiplexers sowie

**Figur 6b** eine schematische Aufsicht auf den Multiplexer zur Veranschaulichung der Funktionsweise.

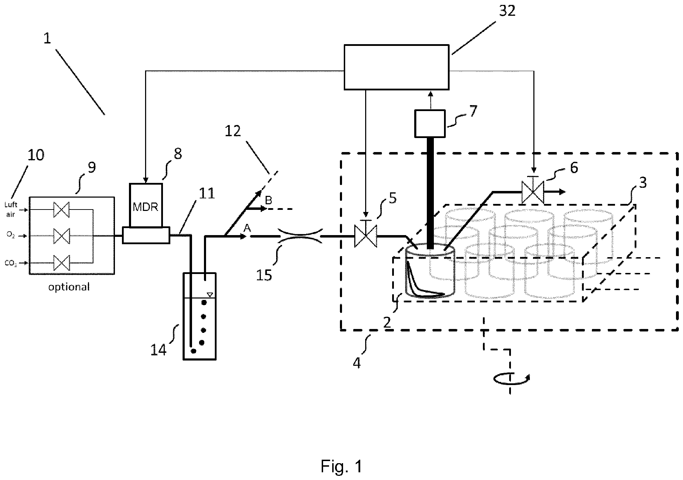

Figur 1 zeigt eine Vorrichtung zur Ermittlung des physiologischen Zustandes von mikrobiellen Kulturen in jedem einzelnen Mikrobioreaktor (2) einer Mikrotiterplatte (3), wobei die Mikrotiterplatte (3) mit einem Schütteltablar (4) in eine Schüttelbewegung versetzt wird. Unter den geschüttelten Bedingungen soll gemäß der Erfindung eine kontrollierte, gleichmäßige Versorgung jedes einzelnen Mikrobioreaktors (2) mit einem Spülgas ermöglicht werden. Mit einer erfindungsgemäßen Vorrichtung kann beispielsweise eine Sauerstofflimitierung in jedem einzelnen Mikrobioreaktor (2) erkannt werden, ohne die gelöste Sauerstoffkonzentration in der mikrobiellen Kultur direkt zu messen.

Die Sauerstofftransferrate (OTR) berechnet sich wie folgt:

$$OTR = \frac{dp_{O2}}{dt} \cdot \frac{Vg}{V_{fl} \cdot R \cdot T} \qquad \text{Formel 1}$$

| | |
|---|---|
| $dpO_2/dt$: | Differentialquotient Sauerstoffpartikeldruck über die Zeit [bar/min] |
| $V_g$: | Gasvolumen des Mikrobiorekators [ml] |
| $V_{fl}$: | Flüssigkeitsvolumen des Mikrobioreaktors [ml] |
| T: | Temperatur [K] |
| R: | Gaskonstante [bar*l/mol/K] |

Aus der Form der Abfallkurve des Sauerstoffpartialdrucks in der Messphase lässt sich entnehmen, ob der Sauerstofftransport von der Sauerstoffverbrauchsgeschwindigkeit der Mikroorganismen (reaktionslimitiert) abhängt oder von dem Stoffübergang (Gasphase zu Flüssigphase) (stoffübergangslimitiert). Im ersten Fall ist der Sauerstoffverbrauch unabhängig von dem treibenden Partialdruckgefälle, d.h. der Differentialquotient aus der Formel 1 kann durch einen Differenzenquotient ersetzt werden:

$$\frac{\Delta po_2}{\Delta t} = OTR \cdot \frac{V_{fl} \cdot R \cdot T}{Vg} \qquad \text{Formel 2}$$

Formel 2 zeigt, dass bei einem linearen Sauerstoffpartialdruckabfall in der Meßphase keine Sauerstofflimitierung der Kultur vorliegt.

Liegt eine Sauerstofflimitierung vor (stoffübergangslimitiert), so ist der Sauerstoffverbrauch nicht mehr unabhängig von dem treibenden Partialdruckgefälle und die Gleichung für den Partialdruckabfall in der Meßphase sieht wie folgt aus:

$$\frac{ln\dfrac{po_{22}}{po_{21}}}{\Delta t} = k_La \cdot \frac{V_{fl} \cdot R \cdot T}{Vg \cdot He} \qquad \text{Formel 3}$$

| | |
|---|---|
| $k_La$: | volumetrischer Stoffdurchgangskoeffizient [1/h] |
| He: | Henry'sehe Konstante [bar*l/mol] |
| $P_{O21}$: | Sauerstoffpartialdruck am Anfang der Meßphase [bar] |
| $P_{O22}$: | Sauerstoffpartialdruck am Ende der Meßphase [bar] |

Diese Abhängigkeit von dem treibenden Partialdruckgefälle führt zu einer nicht linearen Kurvenform.

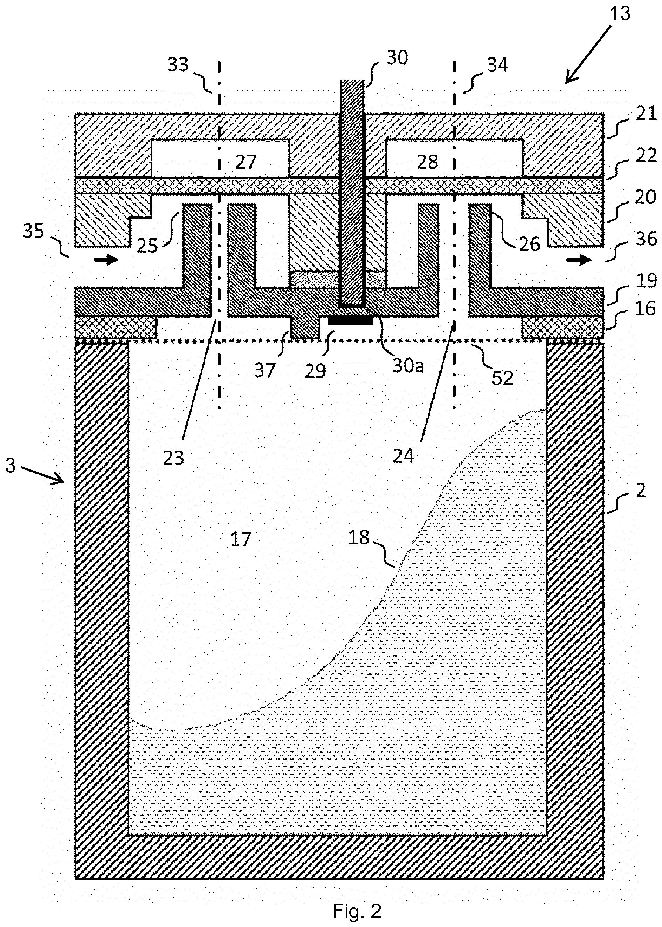

Jeder Mikrobioreaktor (2) verfügt über ein Einlass- und ein Auslassventil (5, 6), die in der Messphase der Vorrichtung geschlossen werden. Sowohl das Einlassventil (5) als auch das Auslassventil (6) sind unmittelbar an jedem Mikrobioreaktor angeordnet, wie dies aus den Abbildungen 2-5 ersichtlich ist. Mithilfe von Messmitteln (7) werden in jedem einzelnen Mikrobioreaktor Änderungen des Partialdrucks des Spülgases erfasst und daraus die Transferraten in einem Messrechner (32) berechnet. Der Messrechner (32) steuert außerdem die Ein- und Auslassventile (5,6), einen Massendurchflussregler (8) und gegebenenfalls eine optionale Gasmischbatterie (9).

Mit einer Einspeisung (10) wird das Spülgas über eine Gasverteilung umfassend eine zentrale Zuführleitung (11) und eine in Figur 1 der Übersichtlichkeit halber nicht dargestellte Unterverteilung (12) den Einzelnen Mikrobioreaktoren (2) zugeführt und aus diesen über die Auslassventile (6) wieder ausgeleitet. Die Unterverteilung (12) ist in einer auf die Mikrotiterplatte (3) aufgesetzten Abdeckung (13) mit weiteren funktionalen Elementen zur Steuerung des Spülgases angeordnet.

In der zentralen Zuführleitung (11) kann zusätzlich eine Waschflache (14) angeordnet sein, um einen Flüssigkeitsverlust durch Verdunstung während der Kultivierung auszugleichen. Um eine übereinstimmende Spülgasversorgung in sämtlichen Mikrobioreaktoren (2) sicher zu stellen, schalten sämtliche Einlassventile (5) und sämtliche Auslassventile (6) der Mikrobioreaktoren (2) jeweils im Kollektiv. Schließlich ist in Figur 1 schematisch mit der Positionsziffer (15) der Strömungswiderstand jedes Mikrobioreaktors (2) angedeutet.

Nachfolgend wird anhand der Teildarstellung in Figur 2 in Verbindung mit Figur 1 der Aufbau der Abdeckung (13) näher erläutert. Zur Veranschaulichung der Integration der Ein- und Auslassventile (5,6) sowie deren pneumatischer Betätigung ist die Darstellung nicht maßstäblich. Zwischen der Abdeckung (13) und der Mikrotiterplatte (3) befindet sich eine Dichtung (16) um einen Gasaustritt aus dem Gasraum (17) oberhalb der mikrobiellen Kultur (18) im geschlossenen Zustand der beiden Ventile (5,6) zu verhindern. Konstruktiv setzt sich die Abdeckung (13) aus einer transparenten Bodenplatte (19), einer Mittelplatte (20) sowie einer Deckelplatte (21) zusammen. Zwischen der Deckelplatte (21) und der Mittelplatte (20) ist eine Schaltmembran (22) angeordnet.

In der Bodenplatte (19) befinden sich Einlassöffnungen (23) und Auslassöffnungen (24), wobei der Gasraum (17) jedes Mikrobioreaktors (2) über eine Einlassöffnung (23) und eine Auslassöffnung (24) zugänglich ist. Des Weiteren umfasst die Bodenplatte (19) einen jede Einlassöffnung (23) umgebenden Ventilsitz (25) sowie einen jede Auslassöffnung (24) umgebenden Ventilsitz (26). Die zwischen der Mittelplatte (20) und der Deckelplatte (21) angeordnete Schaltmembran (22) begrenzt bodenseitig erste Druckkammern (27) und zweite Druckkammern (28). Abhängig von dem steuerbaren, innerhalb der Druckkammern (27,28) herrschenden Druck setzt sich die elastische Schaltmembran (22) auf die Ventilsitze (25,26) und verschließt die Einlass- beziehungsweise Auslassventile (5,6) im Kollektiv. Die in die Bodenplatte (19) integrierten Ventilsitze (25, 26) bilden zusammen mit der ebenfalls in die Abdeckung (13) integrierten Schaltmembran (22) sämtliche Absperrmittel zum Unterbrechen des Spülgasstroms in die Mikrobioreaktoren (2).

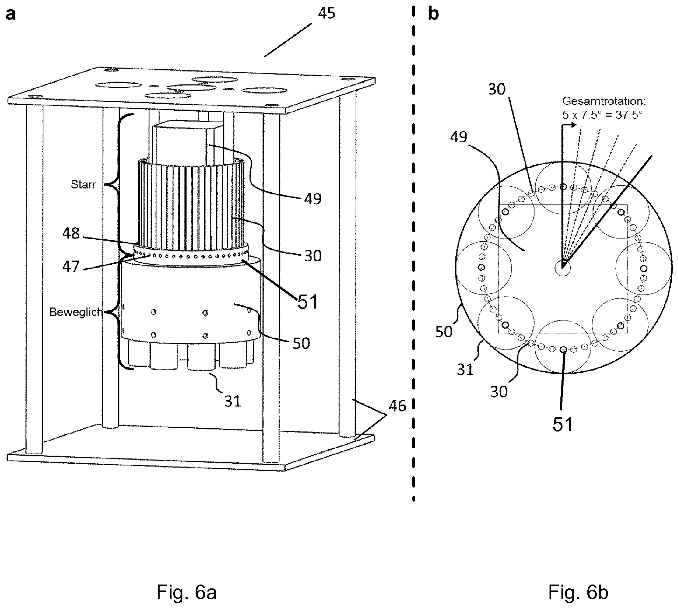

Weiter lässt sich aus Figur 2 entnehmen, wie die in Figur 1 lediglich schematisch dargestellten Messmittel(7) teilweise in die Abdeckung (13) integriert sind. An der zum Gasraum (17) jedes Mikrobioreaktors (2) weisenden Oberfläche der transparenten Bodenplatte (19) ist ein passives Messelement (29) angeordnet. Bei dem passiven Messelement (29) handelt es sich beispielsweise um einen partialdrucksensitiven Fluoreszenzspot. Auf Änderungen der Gaskonzentration im Gasinnenraum (17) reagiert der Fluoreszenzspot mit einer Änderung der ausgesendeten elektromagnetischen Strahlung, die über eine Übertragungsleitung (30) von jedem Mikrobioreaktor (2) kontaktlos zu einem in Figur 6a, 6b dargestellten Messwertgeber (31) übertragen wird. Die Übertragungsleitung (30), beispielsweise in Form eines Lichtwellenleiters, ist endseitig auf der dem passiven Messelement (29) gegenüberliegenden Seite in eine Einlassung (30a) der transparenten Abdeckung (19) eingebracht.

Die Positionsziffer (35) zeigt schließlich einen Teil, der das Spülgas zuführenden Leitungsstruktur (35) der Unterverteilung (12) und die Positionsziffer (36) einen Teil der das Spülgas abführenden Leitungsstruktur der Unterverteilung (12). Darüber hinaus ist eine strömungsleitende Barriere (37) an der Unterseite der Bodenplatte (19) zwischen der Einlassöffnung (23) und der Auslassöffnung (24) jedes Mikrobiorekators (2)angeordnet, die eine Kurzschlussströmung des Spülgases bei geöffneten Ventilen unmittelbar zwischen der Einlass- und der Auslassöffnung (23,24) verhindert.

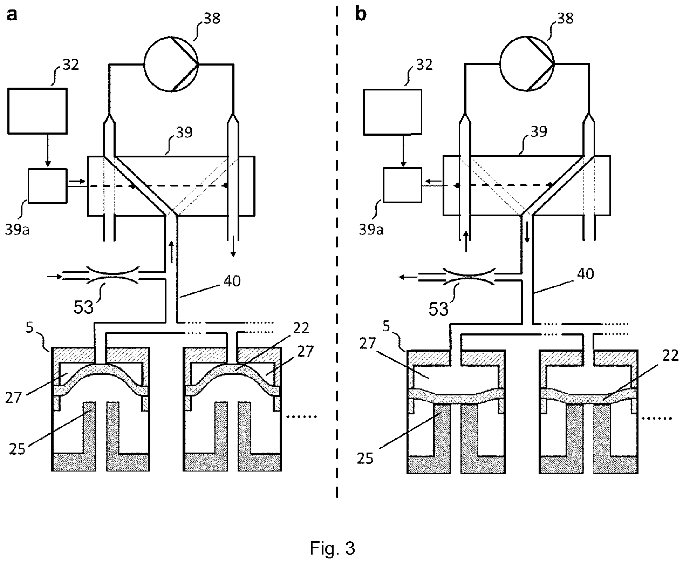

Nachfolgend wird anhand der Figuren 3 a, b die von dem Messrechner (32) kontrollierte Betätigung der Einlassventile (5) anhand einer Darstellung eines Ausschnitts der Abdeckung (13) näher erläutert. Zur Erzeugung eines Unter- oder Überdrucks in einer ersten Druckkammer (27) ist eine elektrisch angetriebene Pumpe (38) vorgesehen, deren Saug- und Druckseite mit den Anschlüssen eines 5/2-Wegeventils (39) verbunden sind. In der in Figur 3 a dargestellten Schaltstellung des 5/2 Wegeventils (39) ist die Saugseite der Pumpe (38) über eine Leitung (40) mit der ersten Druckkammer (27) verbunden. Durch den Unterdruck wird die elastische Schaltmembran (22) in Richtung der Oberseite der ersten Druckkammer (27) gezogen. Der Unterdruck wird während des geöffneten Zustandes der Einlassventile (5) aufrecht erhalten, um eine kontrollierte Schaltstellung zu gewährleisten. In der in Figur 3b dargestellten Schaltstellung des 5/2 Wegeventils (39) ist die erste Druckkammer (27) fluidleitend mit der Druckseite der Pumpe (38) verbunden. Der nunmehr in der ersten Druckkammer (27) herrschende Überdruck drückt die elastische Schaltmembran (22) auf den Ventilsitz (25) jedes Einlassventils (5).

An die Leitung (40) ist eine Stichleitung mit einer Drossel (53) angeschlossen, die im Schaltzustand des Einlassventils (5) nach Figur 3 a den Unterdruck und im Schaltzustand des Einlassventils (5) nach Figur 3 b den Überdruck begrenzt.

Zum Schalten des 5/2 Wegeventils (39) ist dieses über einen Aktuator (39a) mit dem Messrechner (32) verbunden. Die Betätigung der Auslassventile (6) mit Hilfe der 2. Druckkammer (28) erfolgt in gleicher Weise, sodass auf eine gesonderte Darstellung der Ventilbetätigung verzichtet wird.

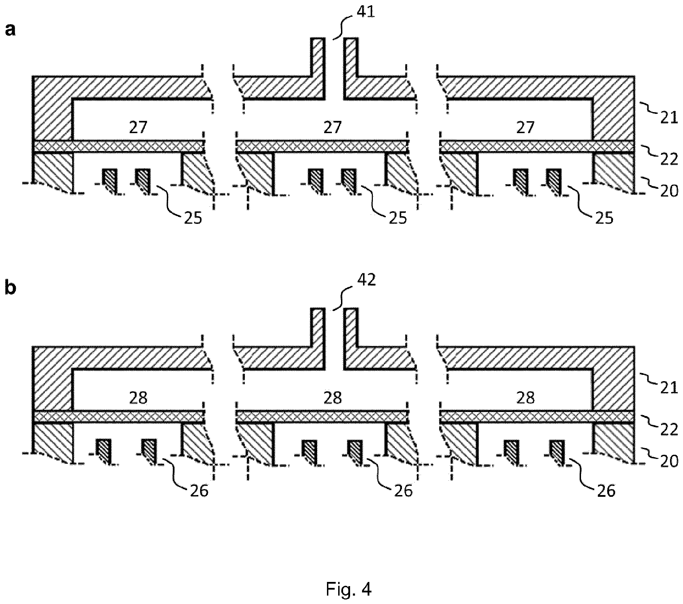

Die in Figuren 4 a, b dargestellten Schnitte entlang der Schnittlinien 33, 34 nach Figur 2 veranschaulichen, wie mehrere in einer Reihe angeordnete Einlassventile (5) über eine gemeinsame erste Druckkammer (27) und mehrere in einer Reihe angeordnete Auslassventile (6) über eine gemeinsame zweite Druckkammer (28) betätigt werden. Hierzu ist jede erste Druckkammer (27) beziehungsweise jede zweite Druckkammer (28) zur Betätigung einer Reihe von Einbeziehungsweise Auslassventilen (5,6) über einen Anschluss (41) beziehungsweise (42) mit der in Figur 3 dargestellten Ventilsteuerung verbunden.

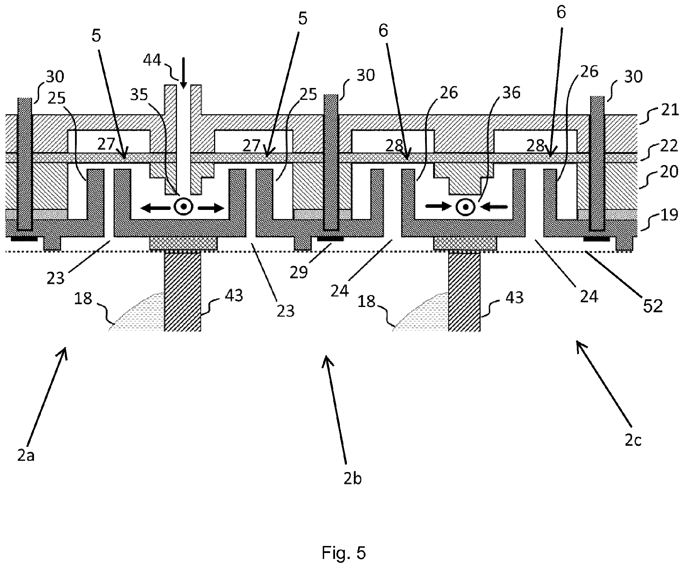

Der in der Schnittrichtung mit Figur 2 übereinstimmende Teilschnitt nach Figur 5 zeigt insgesamt drei Mikrobioreaktoren (2a, 2b, 2c) zur Veranschaulichung des Aufbaus der Unterverteilung (12) für das Spülgas innerhalb der Abdeckung (13). Jeder Mikrobiorekator (2a, 2b, 2c) ist Bestandteil einer senkrecht zur Bildebene verlaufenden Reihe von Mikrobioreaktoren, die sämtlich durch Trennwände (43) voneinander getrennt sind. Es ist deutlich erkennbar, wie die Einlassventile (5) der benachbarten Reihen mit Mikrobioreaktoren (2a, 2b) über die zuführende Leitungsstruktur (35) im geöffneten Zustand der Ventile mit Spülgas versorgt werden. Des Weiteren ist erkennbar, wie die Auslassventile (6) der benachbarten Reihen mit Mikrobioreaktoren (2b, 2c) in fluidleitender Verbindung mit der abführenden Leitungsstruktur (36) zur Ausleitung des Spülgases aus den Mikrobioreaktoren stehen.

An den in der zuführenden Leitungsstruktur (35) mündenden Anschluss (44) ist die in Figur 1 dargestellte zentrale Zuführleitung (11) der Gasversorgung angeschlossen. Durch in der Schnittdarstellung nach Figur 5 nicht sichtbare weitere Leitungsstrukturen der Unterverteilung (12) gelangt das über den Anschluss (44) zugeführte Spülgas zu weiteren, in Figur 5 nicht dargestellten Reihen mit Einlassventilen (5).

Die Länge der zu- und abführenden Leitungsstruktur (35, 36) zu jedem einzelnen Mikrobioreaktor (2a, 2b, 2c) ist unterschiedlich. Eine gleichmäßige Spülgasversorgung sämtlicher Mirkobioreaktoren wird gleichwohl gewährleistet, weil die kumulierten Strömungswiderstände in der Gasversorgung bis zu dem jeweiligen Mikrobiorekator (2a, 2b, 2c), d.h. in der Zuführleitung (11) und Unterverteilung (12) gegenüber dem Strömungswiderstand in dem jeweiligen Mikrobioreaktor (2a, 2b, 2c) vernachlässigbar sind. Infolgedessen genügt für die gleichmäßige Versorgung sämtlicher Mikrobioreaktoren mit Spülgas ein einziges flusssteuerndes Bauteil, beispielsweise der in Figur 1 dargestellte Massendurchflussregler (8).

Die Atmungsaktivitäten der mikrobiellen Kulturen führt zu einer Änderung der Spülgaskonzentration in dem geschlossenen Gasinnenraum (17) jedes Mikrobioreaktors (2) und geht mit einer Änderung der von dem Fluoreszenzspot ausgesendeten elektromagnetischen und in den Lichtwellenleiter eingekoppelten Strahlung einher. Die Lichtwellenleiter werden von der Mikrotiterplatte (3) zu einem neben dem in Figur 1 dargestellten Schütteltablar (4) aufstellbaren optischen Multiplexer (45) geleitet, der in Figur 6a dargestellt ist. Der optische Multiplexer (45) umfasst einen Rahmen (46) mit einem ortsfest gehalterten Ringkörper (47), an dem eine der Anzahl der Übertragungsleitungen (30) (Lichtwellenleiter) entsprechende Anzahl von ersten Anschlüssen (48) angeordnet ist. Des Weiteren ist an dem Rahmen (46) ein Drehantrieb (49), beispielsweise in Form eines Schrittmotors, befestigt, dessen Abtriebswelle mit einer zylindrischen Halterung (50) verbunden ist. Die Drehachse der Halterung (50) verläuft koaxial zu der Achse des Ringkörpers (47). An der Halterung (50) sind zweite Anschlüsse (51) des Multiplexers derart angeordnet, dass diese mit den ersten Anschlüssen (48) durch Verdrehen der Halterung (50) in eine für die Messignale sowie das Anregungslicht der modulierbaren Lichtquelle leitende Verbindung bringbar sind. An jeden zweiten Anschluss (51) ist einer der optoelektronischen Messwertgeber (31) angeschlossen. Die Anzahl der passiven Messelemente (29) ist um ein ganzzahliges Vielfaches größer als die Anzahl der Messwertgeber (31).

Durch Betätigen des Drehantriebs können die elektromagnetischen Signale an den ersten Anschlüssen (48) nacheinander an einen der zweiten Anschlüsse (51) durchgeschaltet und auf diese Weise einem der Messwertgeber (31) zugeführt werden, der die von den einzelnen Mikrobioreaktoren zugeführte elektromagnetische Strahlung in elektrische Signale wandelt. Des weiteren wird das in den Messwertgebern (31) generierte Anregungslicht an den zweiten Anschlüssen (51) an einen der ersten Anschlüsse (48) durchgeschaltet und auf diese Weise einem der passiven Messelemente (29) zuvor zugeführt.

## Bezugszeichenliste

| Nr. | Bezeichnung | Nr. | Bezeichnung |
|---|---|---|---|
| 1. | Vorrichtung | 29. | passives Messelement |
| 2.a,b,c | Mikrobioreaktoren | 30. | Übertragungsleitung |
| 3. | Mikrotiterplatte | 30a. | Einlassung |
| 4. | Schütteltablar | 31. | Messwertgeber |
| 5. | Einlassventil | 32. | Messrechner |
| 6. | Auslassventil | 33. | Schnittlinie |
| 7. | Messmittel | 34. | Schnittlinie |
| 8. | Massendurchflussregler | 35. | zuführende Leitungsstruktur |
| 9. | Gasgemischbatterie | 36. | abführende Leitungsstruktur |
| 10. | Einspeisung (Spülgas) | 37. | Barriere |
| 11. | zentrale Zuführleitung | 38. | Pumpe |
| 12. | Unterverteilung | 39. | 5/2 Wegeventil |
| 13. | Abdeckung | 39a. | Aktuator |
| 14. | Waschflasche | 40. | Anschluss |
| 15. | Strömungswiderstand | 41. | Anschluss 1. Druckkammer |
| 16. | Dichtung | 42. | Anschluss 2. Druckkammer |
| 17. | Gasraum | 43. | Trennwände |
| 18. | mikrobielle Kultur | 44. | Anschluss |
| 19. | Bodenplatte | 45. | optischer Multiplexer |

(fortgesetzt)

| Nr. | Bezeichnung | Nr. | Bezeichnung |
|-----|-------------|-----|-------------|
| 20. | Mittelplatte | 46. | Rahmen |
| 21. | Deckelplatte | 47. | Ringkörper |
| 22. | Schaltmembran | 48. | Erster Anschluss |
| 23. | Einlassöffnung | 49. | Drehantrieb |
| 24. | Auslassöffnung | 50. | Halterung |
| 25. | Ventilsitz (Einlassventil) | 51. | Zweiter Anschluss |
| 26. | Ventilsitz (Auslassventil) | 52. | Sterilbarriere |
| 27. | 1. Druckkammer | 53. | Drossel |
| 28. | 2. Druckkammer | | |

**Patentansprüche**

1. Vorrichtung (1) zur Ermittlung und Überwachung des physiologischen Zustandes von mikrobiellen Kulturen (18) in jedem einzelnen Mikrobioreaktor (2) einer Mikrotiterplatte (3), wobei ein Gasraum (17) jedes Mikrobioreaktors (2) über eine Einlass- und Auslassöffnung (23, 24) zugänglich ist, umfassend

   - Mittel zum Schütteln der Mikrotiterplatte, wobei die Mittel einen Schüttler und ein Schütteltablar (4) aufweisen,
   - eine Gasversorgung geeignet um den Gasraum (17) jedes Mikrobioreaktors (2) in einer Spülphase mit einem Spülgasstrom zu spülen, die eine Spülgaseinspeisung (10) und eine Gasverteilung (11,12) aufweist,
   - ein unmittelbar an jedem Mikrobioreaktor (2) angeordnetes Absperrmittel geeignet zum Unterbrechen des Spülgasstroms, wobei die Absperrmittel ein Einlassventil aufweisen, das einen die Einlassöffnung (23) umgebenden Ventilsitz (25) sowie eine pneumatisch betätigte Absperrmembran (22) zum Öffnen und Schließen der Einlassöffnung (23) umfasst, wobei die Einlassöffnung (23) zugleich den Strömungswiderstand jedes Mikrobioreaktors bildet,

     - wobei die zum Zuführen des eingespeisten Spülgases und zum Abführen des Spülgases von jedem Mikrobioreaktor (2) eingerichtete Gasverteilung (11, 12) eine zentrale Zuführleitung (11) aufweist, die sich von der Spülgaseinspeisung (10) zu einer an der Mikrotiterplatte angeordneten Unterverteilung (12) für das Spülgas erstreckt und
     - die Strömungswiderstände in der Gasversorgung und der Strömungswiderstand jedes Mikrobioreaktors (2) derart eingerichtet sind, dass der Spülgasstrom in der Spülphase in sämtlichen Mikrobioreaktoren (2) im technischen Sinne übereinstimmt, sowie

   - Messmittel(7), die zur Erfassung des physiologischen Zustandes der mikrobiellen Kultur in jedem einzelnen Mikrobioreaktor (2) in einer Meßphase bei unterbrochenem Spülgasstrom eingerichtet sind.

2. Vorrichtung nach Anspruch 1, **dadurch gekennzeichnet, dass** die Gasversorgung ein in der zentralen Zuführleitung (11) angeordnetes flusssteuerndes Bauteil (8) aufweist.

3. Vorrichtung nach Anspruch 1, **dadurch gekennzeichnet, dass** die Gasversorgung eine in der zentralen Zuführleitung (11) angeordnete Waschflasche (14) aufweist.

4. Vorrichtung nach einem der Ansprüche 1 bis 3, **dadurch gekennzeichnet, dass** die Absperrmittel außerdem ein Auslassventil aufweisen, das einen die Auslassöffnung(24) umgebenden Ventilsitz (25) sowie eine pneumatisch betätigte Absperrmembran (22) zum Öffnen und Schließen der Auslassöffnung (24) umfasst.

5. Vorrichtung nach einem der Ansprüche 1 bis 4, **dadurch gekennzeichnet, dass** auf der dem Ventilsitz (25) abgewandten Seite der Absperrmembran (22) mehrerer Einlassventile eine mit Unter- und/oder Überdruck beaufschlagbare Druckkammer (27) angeordnet ist, die zum gleichzeitigen pneumatischen Betätigen der Absperrmembran (22) mehrerer Einlassventile eingerichtet ist.

**6.** Vorrichtung nach Anspruch 4 oder 5, **dadurch gekennzeichnet, dass** auf der dem Ventilsitz (26) abgewandten Seite der Absperrmembran (22) mehrerer Auslassventile eine mit Unter- und/oder Überdruck beaufschlagbare Druckkammer (28) angeordnet ist, die zum gleichzeitigen pneumatischen Betätigen der Absperrmembran (22) mehreren Auslassventile eingerichtet ist.

**7.** Vorrichtung nach einem der Ansprüche 1 bis 6, **dadurch gekennzeichnet, dass** die Absperrmittel sämtlicher Mikrobioreaktoren (2) übereinstmmen.

**8.** Vorrichtung nach einem der Ansprüche 1 bis 7, **dadurch gekennzeichnet, dass** der Strömungswiderstand jedes Mikrobioreaktors (2) in der Spülphase mindestens um den Faktor 100 höher ist als die Strömungswiderstände der Gasverteilung (11, 12) bis zu dem jeweiligen Mikrobioreaktor (2).

**9.** Vorrichtung nach Anspruch 8, **dadurch gekennzeichnet, dass** die Strömungswiderstände sämtlicher Mikrobioreaktoren (2) übereinstimmen.

**10.** Vorrichtung nach einem der Ansprüche 1 bis 9, **dadurch gekennzeichnet, dass** die Auslassöffnung (24) zugleich den Strömungswiderstand jedes Mikrobioreaktors (2) bildet.

**11.** Vorrichtung nach einem der Ansprüche 1 bis 10, **dadurch gekennzeichnet, dass** der Querschnitt der Einlassöffnung (23) jedes Mikrobioreaktors (2) kleiner als der Querschnitt der Auslassöffnung (24) jedes Mikrobioreaktors (2) ist.

**12.** Vorrichtung nach einem der Ansprüche 1 bis 11, **dadurch gekennzeichnet, dass** die Messmittel (7) zur Erfassung mindestens einer für die Atmungsaktivität aussagekräftigen Größe der mikrobiellen Kultur zumindest teilweise in jedem Mikrobioreaktor angeordnet sind.

**13.** Vorrichtung nach einem der Ansprüche 1 bis 12, **dadurch gekennzeichnet, dass** die Messmittel folgendes umfassen:

- mindestens ein in jedem Mikrobioreaktor (2) angeordnetes passives Messelement (29), dessen Messsignal sich durch eine Änderung der Atmungsaktivität ändert,
- Meßwertgeber (31) zum Umformen des Messsignals in elektrische Signale sowie
- Übertragungsleitungen (30) zum Übertragen des Messignals zwischen jedem passiven Messelement (29) und einem der Meßwertgeber (31).

**14.** Vorrichtung nach Anspruch 13, **dadurch gekennzeichnet, dass** jedes passive Messelement (29) eine an einer transparenten Fläche des Mikrobioreaktors (2) dauerhaft angeordnete Indikatorschicht ist, die auf Änderungen der Gaskonzentration im Gasinnenraum mit einer Änderung der ausgesendeten elektromagnetischen Strahlung reagiert und dass jeder Messwertgeber (31) als optoelektronisches Bauelement ausgebildet ist.

**15.** Vorrichtung nach Anspruch 13 oder 14, **dadurch gekennzeichnet, dass**

- die Anzahl der passiven Messelemente (29) und die Anzahl der Übertragungsleitungen (30) um ein ganzzahliges Vielfaches größer als die Anzahl der Messwertgeber (31) ist und
- die Übertragungsleitungen (30) mit ersten Anschlüssen eines optischen Multiplexers (45) und die Messwertgeber (31) mit zweiten Anschlüssen des optischen Multiplexers (45) verbunden sind, wobei an unterschiedlichen ersten Anschlüssen (48) anliegende Messignale nacheinander an einen der zweiten Anschlüsse (51) durchschaltbar sind.

**16.** Vorrichtung nach einem der Ansprüche 13 bis 15, **dadurch gekennzeichnet, dass** jeder Meßwertgeber (31) eine modulierbare Lichtquelle und einen optoelektronischen Sensor umfasst und die Lichtquellen sämtlicher Meßwertgeber (31) unterschiedliche Modulationsfrequenzen aufweisen.

**17.** Vorrichtung nach Anspruch einem der Ansprüche 1 bis 16, **dadurch gekennzeichnet, dass** sämtliche Absperrmittel in eine Abdeckung (13) integriert sind, die auf die Mikrotiterplatte (3) aufsetzbar ist.

**18.** Vorrichtung nach Anspruch 17, **dadurch gekennzeichnet, dass** die Unterverteilung (12) für das Spülgas in die Abdeckung (13) integriert ist.

**19.** Vorrichtung nach einem der Ansprüche 17 bis 18, **dadurch gekennzeichnet, dass** die mit Unter- und/oder Überdruck beaufschlagbaren Druckkammern (27, 28) zum pneumatischen Betätigen der Absperrmembran (22) in die Abdeckung (13) integriert sind.

**20.** Vorrichtung nach Anspruch einem der Ansprüche 17 bis 19, **dadurch gekennzeichnet, dass** zwischen der Mikrotiterplatte (2) und der Abdeckung (13) eine Sterilbarriere (52) angeordnet ist.

**21.** Vorrichtung nach einen der Ansprüche 1 bis 20, **dadurch gekennzeichnet, dass** zwischen der Einlass- und Auslassöffnung (23,24) eine Barriere (29) derart angeordnet ist, dass ein Kurzschluss des Spülgasstroms zwischen Ein- und Auslass unterbunden ist.

**22.** Vorrichtung nach einen der Ansprüche 17 bis 21, **dadurch gekennzeichnet, dass** die Abdeckung (13) aus mehreren miteinander verbundenen Platten zusammengesetzt ist.

**Claims**

**1.** A device (1) for determining and monitoring the physiological state of microbial cultures (18) in each individual microbioreactor (2) of a microtitre plate (3), wherein a gas space (17) of each microbioreactor (2) is accessible via an inlet and outlet opening (23, 24), comprising:

- means for shaking the microtitre plate, wherein the means comprise a shaker and a shaker tray (4),
- a gas supply system suitable for purging the gas space (17) of each microbioreactor (2) with a stream of purge gas in a purging phase, which comprises a purge gas feed-in (10) and a gas distribution system (11, 12),
- a shut-off means arranged directly on each microbioreactor (2) and suitable for interrupting the stream of purge gas, wherein the shut-off means comprise an inlet valve comprising a valve seat (25) surrounding the inlet opening (23) as well as a pneumatically actuated shut-off membrane (22) for opening and closing the inlet opening (23), wherein the inlet opening (23) at the same time forms the flow resistance of each microbioreactor,

- wherein the gas distribution system (11, 12) adapted to deliver the fed-in purge gas and to remove the purge gas from each microbioreactor (2) has a central delivery line (11) which extends from the purge gas feed-in (10) to a subdistributor (12) for the purge gas arranged on the microtitre plate and
- the flow resistances in the gas supply system and the flow resistance of each microbioreactor (2) are adapted in such a manner that the stream of purge gas in the purging phase is substantially equal in the technical sense in all the microbioreactors (2), and

- a measuring device (7) adapted to detect the physiological state of the microbial culture in each individual microbioreactor (2) in a measuring phase whilst the stream of purge gas is interrupted.

**2.** The device according to claim 1, **characterized in that** the gas supply system comprises a flow-controlling component (8) arranged in the central delivery line (11).

**3.** The device according to claim 1, **characterized in that** the gas supply system comprises a wash bottle (14) arranged in the central delivery line (11).

**4.** The device according to one of claims 1 to 3, **characterized in that** the shut-off device additionally comprises an outlet valve which comprises a valve seat (25) surrounding the outlet opening (24) and a pneumatically actuated shut-off membrane (22) for opening and closing the outlet opening (24).

**5.** The device according to one of claims 1 to 4, **characterized in that** a pressure chamber (27) which can be acted upon by underpressure and/or overpressure, and which is adapted for simultaneous pneumatic actuation of the shut-off membrane (22) of several inlet valves, is arranged on the side of the shut-off membrane (22) of several inlet valves facing away from the valve seat (25).

**6.** The device according to claim 4 or 5, **characterized in that** a pressure chamber (28) which can be acted upon by underpressure and/or overpressure, and which is adapted for simultaneous pneumatic actuation of the shut-off membrane (22) of several outlet valves, is arranged on the side of the shut-off membrane (22) of several outlet valves facing away from the valve seat (26).

7. The device according to one of claims 1 to 6, **characterized in that** the shut-off devices of all the microbioreactors (2) are identical.

8. The device according to one of claims 1 to 7, **characterized in that** the flow resistance of each microbioreactor (2) in the purging phase is higher than the flow resistances of the gas distribution system (11, 12) as far as the respective microbioreactor (2) by at least a factor of 100.

9. The device according to claim 8, **characterized in that** the flow resistances of all the microbioreactors (2) are equal.

10. The device according to one of claims 1 to 9, **characterized in that** the outlet opening (24) at the same time determines the flow resistance of each microbioreactor (2).

11. The device according to one of claims 1 to 10, **characterized in that** the cross-section of the inlet opening (23) of each microbioreactor (2) is smaller than the cross-section of the outlet opening (24) of each microbioreactor (2).

12. The device according to one of claims 1 to 11, **characterized in that** the measuring device (7) for detecting at least one parameter of the microbial culture representative of the respiration activity is arranged at least partially in each microbioreactor.

13. The device according to one of claims 1 to 12, **characterized in that** the measuring device comprises the following:

    - at least one passive measuring element (29) arranged in each microbioreactor (2), the measurement signal of which changes as a result of a change of respiration activity,
    - transducers (31) for converting the measurement signals to electrical signals, and
    - transmission lines (30) for transmitting the measurement signal between each passive measuring element (29) and one of the transducers (31).

14. The device according to claim 13, **characterized in that** each passive measuring element (29) is an indicator layer arranged permanently on a transparent surface of the microbioreactor (2) which reacts to changes of the gas concentration in the gas interior with a change in the emitted electromagnetic radiation, and that each transducer (31) is configured as an optoelectronic component.

15. The device according to claim 13 or 14, **characterized in that**

    - the number of passive measuring elements (29) and the number of transmission lines (30) is greater than the number of transducers (31) by an integral multiple, and
    - the transmission lines (30) are connected to first ports of an optical multiplexer (45) and the transducers (31) are connected to second ports of the optical multiplexer (45), wherein measurement signals lying at different first ports (48) can be switched through in succession to one of the second ports (51).

16. The device according to one of claims 13 to 15, **characterized in that** each transducer (31) comprises a modulatable light source and an optoelectronic sensor, and the light sources of all the transducers (31) have different modulation frequencies.

17. The device according to one of claims 1 to 16, **characterized in that** all the shut-off devices are integrated in a cover (13) that can be fitted onto the microtitre plate (3).

18. The device according to claim 17, **characterized in that** the subdistributor (12) for the purge gas is integrated in the cover (13).

19. The device according to one of claims 17 to 18, **characterized in that** the pressure chambers (27, 28) which can be acted upon by underpressure and/or overpressure, and which are provided for pneumatic actuation of the shut-off membrane (22), is integrated in the cover (13).

20. The device according to one of claims 17 to 19, **characterized in that** a sterile barrier (52) is arranged between the microtitre plate (2) and the cover (13).

21. The device according to one of claims 1 to 20, **characterized in that** a barrier (29) is arranged between the inlet

## EP 3 239 285 B1

and outlet opening (23, 24) in such a manner that a short circuit of the stream of purge gas between inlet and outlet is suppressed.

**22.** The device according to one of claims 17 to 21, **characterized in that** the cover (13) is composed of a plurality of interconnected plates.

## Revendications

**1.** Dispositif (1), destiné à déterminer et à superviser l'état physiologique de cultures microbiennes (18) dans chaque micro-bioréacteur (2) individuel d'une plaque de microtitration (3), un espace gazeux (17) de chaque micro-bioréacteur (2) étant accessible par l'intermédiaire d'un orifice d'entrée et de sortie (23, 24), comprenant

- des moyens destinés à agiter la plaque de microtitration, les moyens comportant un secoueur et un plateau à secousses (4),
- une arrivée de gaz, apte à purger l'espace gazeux (17) de chaque micro-bioréacteur (2) dans une phase de purge à l'aide d'un flux de gaz de purge, qui comporte une alimentation de gaz de purge (10) et une distribution de gaz (11,12),
- un moyen d'arrêt, placé directement sur chaque micro-bioréacteur (2), apte à interrompre le flux de gaz de purge, les moyens d'arrêt comportant une soupape d'admission qui comprend un siège de soupape (25) qui entoure l'orifice d'entrée (23), ainsi qu'une membrane d'arrêt (22) à actionnement pneumatique, destinée à ouvrir et à fermer l'orifice d'entrée (23), l'orifice d'entrée (23) créant simultanément la résistance à l'écoulement de chaque micro-bioréacteur,

- la distribution de gaz (11, 12) agencée pour amener le gaz de purge alimenté et pour évacuer le gaz de purge de chaque micro-bioréacteur (2) comportant un conduit d'amenée (11) central qui s'étend de l'alimentation de gaz de purge (10) vers une subdivision (12) pour le gaz de purge, placée sur la plaque de microtitration
- les résistances à l'écoulement dans l'arrivée de gaz et la résistance à l'écoulement de chaque micro-bioréacteur (2) étant agencées de telle sorte, que pendant la phase de purge, le flux de gaz de purge dans l'ensemble des micro-bioréacteurs (2) soit coïncidant dans le sens technique,

- des moyens de mesure (7), qui sont agencés pour détecter l'état physiologique de la culture microbienne dans chaque micro-bioréacteur (2) individuel, pendant une phase de mesure lorsque le flux de gaz de purge est interrompu.

**2.** Dispositif selon la revendication 1, **caractérisé en ce que** l'arrivée de gaz comporte un élément constitutif (8) contrôleur de flux, placé dans le conduit d'amenée (11) central.

**3.** Dispositif selon la revendication 1, **caractérisé en ce que** l'arrivée de gaz comporte un barboteur (14) placé dans le conduit d'amenée (11) central.

**4.** Dispositif selon l'une quelconque des revendications 1 à 3, **caractérisé en ce que** les moyens d'arrêt comportent en outre une soupape d'échappement qui comprend un siège de soupape (25) entourant l'orifice de sortie (24), ainsi qu'une membrane d'arrêt (22) à actionnement pneumatique, destinée à ouvrir et à fermer l'orifice de sortie (24).

**5.** Dispositif selon l'une quelconque des revendications 1 à 4, **caractérisé en ce que** sur le côté de la membrane d'arrêt (22) qui est opposé au siège de soupape (25) de plusieurs soupapes d'admission est placée une chambre de compression (27) susceptible d'être soumise à une dépression et/ou à une surpression, qui est agencée pour l'actionnement pneumatique simultané de la membrane d'arrêt (22) de plusieurs soupapes d'admission.

**6.** Dispositif selon la revendication 4 ou 5, **caractérisé en ce que** sur le côté de la membrane d'arrêt (22) qui est opposé au siège de soupape (26) de plusieurs soupapes d'échappement est placée une chambre de compression (28) qui est agencée pour l'actionnement pneumatique simultané de la membrane d'arrêt (22) de plusieurs soupapes d'échappement

**7.** Dispositif selon l'une quelconque des revendications 1 à 6, **caractérisé en ce que** les moyens d'arrêt de l'ensemble des micro-bioréacteurs (2) coïncident.

**8.** Dispositif selon l'une quelconque des revendications 1 à 7, **caractérisé en ce que** pendant la phase de purge, la résistance à l'écoulement de chaque micro-bioréacteur (2) est supérieure au moins du coefficient 100 aux résistances à l'écoulement de la distribution de gaz (11, 12) jusqu'au micro-bioréacteur (2) concerné.

**9.** Dispositif selon la revendication 8, **caractérisé en ce que** les résistances à l'écoulement de l'ensemble des micro-bioréacteurs (2) coïncident.

**10.** Dispositif selon l'une quelconque des revendications 1 à 9, **caractérisé en ce que** l'orifice de sortie (24) créé simultanément la résistance à l'écoulement de chaque micro-bioréacteur (2).

**11.** Dispositif selon l'une quelconque des revendications 1 à 10, **caractérisé en ce que** la section transversale de l'orifice d'entrée (23) de chaque micro-bioréacteur (2) est inférieure à la section transversale de l'orifice de sortie (24) de chaque micro-bioréacteur (2).

**12.** Dispositif selon l'une quelconque des revendications 1 à 11, **caractérisé en ce que** les moyens de mesure (7) destinés à détecter au moins une grandeur de la culture microbienne, révélatrice de l'activité respiratoire sont placés au moins en partie dans chaque micro-bioréacteur.

**13.** Dispositif selon l'une quelconque des revendications 1 à 12, **caractérisé en ce que** les moyens de mesure comprennent ce qui suit :

- au moins un élément de mesure passif (29), placé dans chaque micro-bioréacteur (2) dont le signal de mesure varie lors d'une variation de l'activité respiratoire,
- des capteurs de mesure (31), destinés à convertir le signal de mesure en des signaux électriques, ainsi
- que des lignes de transmission (30), destinées à transmettre le signal de mesure entre chaque élément de mesure passif (29) et l'un des capteurs de mesure (31).

**14.** Dispositif selon la revendication 13, **caractérisé en ce que** chaque élément de mesure passif (29) est une couche indicatrice placée de manière durable sur une surface transparente du micro-bioréacteur (2), qui réagit à des variations de la concentration gazeuse dans l'espace intérieur gazeux par une variation du rayonnement électromagnétique émis et **en ce que** chaque capteur de mesure (31) est conçu sous la forme d'un élément constitutif optoélectronique.

**15.** Dispositif selon la revendication 13 ou 14, **caractérisé en ce que**

- le nombre des éléments de mesure passifs (29) et le nombre des lignes de transmission (30) est supérieur d'un multiple entier au nombre des capteurs de mesure (31) et
- les lignes de transmission (30) sont connectées sur des premières bornes d'un multiplexeur optique (45) et les capteurs de mesure (31) sont connectés sur des deuxièmes bornes du multiplexeur optique (45), des signaux de mesure appliqués sur différentes premières bornes (48) pouvant être transférés successivement sur l'une des deuxièmes bornes (51).

**16.** Dispositif selon l'une quelconque des revendications 13 à 15, **caractérisé en ce que** chaque capteur de mesure (31) comprend une source lumineuse modulable et un capteur optoélectronique et **en ce que** les sources lumineuses de l'ensemble des capteurs de mesure (31) présentent des fréquences de modulation différentes.

**17.** Dispositif selon l'une quelconque des revendications 1 à 16, **caractérisé en ce que** tous les moyens d'arrêt sont intégrés dans un couvercle (13) qui peut se poser sur la plaque de microtitration (3).

**18.** Dispositif selon la revendication 17, **caractérisé en ce que** la subdivision (12) du gaz de purge est intégrée dans le couvercle (13).

**19.** Dispositif selon l'une quelconque des revendications 17 à 18, **caractérisé en ce que** les chambres de compression (27, 28) susceptibles d'être soumises à une dépression et/ou à une surpression pour l'actionnement pneumatique de la membrane d'arrêt (22) sont intégrées dans le couvercle (13).

**20.** Dispositif selon l'une quelconque des revendications 17 à 19, **caractérisé en ce qu'**entre la plaque de microtitration (2) et le couvercle (13) est placée une barrière stérile (52).

**21.** Dispositif selon l'une quelconque des revendications 1 à 20, **caractérisé en ce qu'**entre l'orifice d'entrée et de sortie (23, 24), une barrière (29) est placée de sorte à éviter un court-circuit du flux de gaz de purge entre l'entrée et la sortie.

**22.** Dispositif selon l'une quelconque des revendications 17 à 21, **caractérisé en ce que** le couvercle (13) est composé de plusieurs plaques assemblées les unes aux autres.

Fig. 1

Fig. 2

Fig. 3

Fig. 4

Fig. 5

a

45

Starr

48
47

Beweglich

31

46

Fig. 6a

b

30

49

Gesamtrotation:
5 x 7.5° = 37.5°

50

31

30

51

Fig. 6b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4415444 C2
- EP 0905229 B1
- US 2007275455 A1
- DE 102014012246 A1